Europäisches Patentamt

(19) European Patent Office

Office européen des brevets

(11) Publication number: **0 008 255**
**B1**

(12) **EUROPEAN PATENT SPECIFICATION**

(45) Date of publication of patent specification: **13.04.83**

(21) Application number: **79400495.2**

(22) Date of filing: **12.07.79**

(51) Int. Cl.³: **C 12 N 7/00,**
**A 61 K 39/245,**
**A 61 K 39/255**

(54) Herpes virus vaccine and its preparation.

(30) Priority: **21.07.78 US 927069**

(43) Date of publication of application:
**20.02.80 Bulletin 80/4**

(45) Publication of the grant of the patent:
**13.04.83 Bulletin 83/15**

(84) Designated Contracting States:
**AT BE CH DE FR GB IT LU NL SE**

(56) References cited:
**US - A - 3 783 098**
**US - A - 3 959 466**
**US - A - 3 981 771**

(73) Proprietor: **MERCK & CO. INC.**
**126, East Lincoln Avenue P.O. Box 2000**
**Rahway New Jersey 07065 (US)**

(72) Inventor: **McAleer, William Joseph**
**717 Marietta Drive**
**Ambler, Pennsylvania 19002 (US)**
Inventor: **Markus, Henry Zvi**
**1517 Thornberry Road**
**Wyncote, Pennsylvania 19095 (US)**

(74) Representative: **Corre, Jacques Denis Paul et al,**
**Cabinet Regimbeau Corre, Paillet, Martin &**
**Schrimpf 26, Avenue Kléber**
**F-75116 Paris (FR)**

Courier Press, Leamington Spa, England.

Herpes virus vaccine and its preparation

Background of the invention

U.S.—A—3,783,098, issued January 1, 1974, discloses and claims a herpes virus vaccine prepared by infecting cells with the cell-associated virus, extracting the virus in the presence of a stabilizing agent, and lyophilizing to form a storage-stable virus useful as a vaccine. Following lyophilization, the virus is reconstituted to commercial practice with a stabilizing diluent. This vaccine has received substantial commercial acceptance, particularly when used to protect poultry against Marek's disease. The necessity of using a stabilizing diluent to reconstitute the lyophilized vaccine, however, increases substantially the cost of the vaccine.

Detailed description of the invention

It has now been discovered that a new formulation process can be used which makes possible the use of distilled water as a diluent for the herpes virus vaccines.

In present commercial practice, Marek's disease vaccine, is formulated with SPGA, before lyophilization. SPGA is a stabilizer described by Bovarnick et al., *J. Bact.* 59:509—522 (1950). The ingredients per liter of this solution are: Sucrose 74.62 g; $KH_2PO_4$ 0.45 g; $K_2HPO_4$ 1.35 g; L-monosodium glutamate 0.956 g; bovine albumin powder fraction V. When resuspended in water, the vaccine is 0.033 mMolar with respect to $KH_2PO_4$ and 0.078 mMolar with respect to $K_2HPO_4$. The pH is as low as 5.0 and is seldom as high as about 5.7.

According to the present invention, the herpes virus vaccine is formulated at the pre-lyophilization stage with a stabilizer which stabilizes the potency of the herpes virus and a buffer system effective to maintain the pH at from 6.8 to 7.2 after reconstitution of the virus with water to use level by addition of substantially 200 volumes of water relative to the prelyophilization volume of the vaccine. When lyophilized in the presence of such a buffer system, the lyophilized virus can be reconstituted with water to form a stable, viable, vaccine. A preferred buffer system is a mixture of $KH_2PO_4$ and $K_2HPO_4$ in quantity sufficient to form on reconstitution about a 0.12 mM solution with respect to $KH_2PO_4$ and about a 0.88 mM with respect to $K_2HPO_4$.

Thus, the invention is applicable to cell-associated herpes virus broadly including live virulent viruses, live attenuated viruses, live virus antigenically related to Marek's disease and killed viruses. By way of illustration, some of the many cell-associated viruses to which the invention applies are the Group B herpes viruses. Some of the preferred cell-associated viruses applicable to the invention are varicella herpes zoster virus, cytomegalovirus, Burkitt lymphoma virus, Lucke tumor virus, turkey-herpes virus and Marek's disease virus.

Although the examples illustrate the invention with Marek's disease vaccine, other cell-associated herpes viruses can be used.

The following examples illustrate the present invention without, however, limiting the same thereto.

Example 1

Ten roller bottles from a production lot of Marek's vaccine (strain ATCC VR584) grown in chick embryo fibroblasts for 4 days at 38.5°C in medium 119-F10 (Gibco) with 5% sterile fetal calf serum are harvested according to standard procedures. The harvested fluid from these roller bottles is split into 2 conical centrifuge bottles and centrifuged for 10 minutes at 1000 RPM and 3—5°C.

The supernatant liquid is drawn off and 20.4 ml of a pH controlled buffer stabilizer (90 ml SPGA with 10 ml 1M potassium phosphate buffer pH 7.8) is added to one bottle to attain a theoretical cell concentration of $20 \times 10^6$ cells. (New product.) Diluent SPGA is added to the other bottle in the same manner. (Old product.)

The contents of each of the two bottles are dispensed into 30 ml vials and batch sonicated for 2 min. at approximately 35 watts and a temperature of 3—5°C. After sonication each sample is dispensed into 3 ml vials (1.1 ml fill) and frozen at −70°C for approximately 6 hours. The vials are lyophilized, sealed and labeled.

Stability of the lyophilized powder is evaluated after storage at 37°C for 3 days and reconstitution with distilled water. Results are summarized in Table 1.

TABLE 1
Effect of stabilizer on storage stability of lyophilized Marek's disease vaccine

| Lyophilized powder composition | Titer PFU/dose Run 1 | Run 2 | Average titer PFU/dose |
|---|---|---|---|
| New product | 1660 | 2090 | 1895 |
| Old product | 500 | 539 | 515 |

Example 2

Ten roller bottles from a production lot of Marek's vaccine (strain ATCC VR584) grown in chick embryo fibroblasts for 4 days at 38.5°C in medium 199-F10 (Gibco) with 5% sterile fetal calf serum are harvested according to standard procedures. The harvested fluid from these roller bottles is split into 2 conical centrifuge bottles and centrifuged for 10 minutes at 1000 RPM and 3—5°C.

The supernatant liquid is drawn off and 20.4 ml of a pH controlled buffer stabilizer (90 ml SPGA with 10 ml 1M sodium phosphate buffer pH 7.8) is added to one bottle to attain a theoretical cell concentration of $20 \times 10^6$ cells. (New product.) Diluent SPGA is added to a separate bottle) in the same manner. (Old product.)

The contents of each of the two bottles are dispensed into 30 ml vials and batch sonicated for 3 minutes at approximately 35 watts and a temperature of 3—5°C. After sonication, each sample is dispensed into 3 ml vials (1.1 ml fill) and frozen at −70°C for approximately 6 hours. The vials are lyophilized, sealed and labeled.

Stability of the lyophilized powder is evaluated after storage at 37°C for 3 days and reconstitution with distilled water. Results are summarized in Table 2.

TABLE 2
Effect of stabilizer on storage stability of lyophilized Marek's disease vaccine

| Lyophilized powder composition | Titer PFU/dose | | Average titer PFU/dose |
|---|---|---|---|
| | Run 1 | Run 2 | |
| New product | 2230 | 1970 | 2100 |
| Old product | 500 | 530 | 515 |

Example 3

One hundred and thirty two roller bottles from a production lot are harvested according to standard procedure. The harvested fluid is dispensed into conical centrifuge bottles and centrifuged for 10 minutes at 1000 RPM at 3—5°C. The supernatant liquid is drawn off and approximately 30 ml of a pH controlled buffered stabilizer (90 ml SPGA+10 ml 1M potassium phosphate buffer pH 7.8) is added to each of the bottles. The final volume is 700 ml. The theoretical cell concentration is calculated to be approximately $15.1 \times 10^6$ cells/ml.

The 700 ml suspension is held in ice bath and sonicated using a Flow Sonicating apparatus. Following sonication, the vaccine is transferred to a metal can and shell frozen in an alcohol freezer and stored in a −70°C freezer for storage.

A week later the can content is thawed in a water bath at 29°C; final dilution is made and dispensed into 3 ml vials (1.1 ml fill). Vials are frozen at −70°C for approximately 6 hours. The vials are lyophilized, sealed and labeled.

Example 4

Ten roller bottles from a production lot of Marek's vaccine (strain ATCC VR584) grown in chick embryo fibroblasts for 4 days at 38.5°C in medium 199-F10 (Gibco) with 5% sterile fetal calf serum are harvested according to standard procedures. The harvested fluid from these roller bottles is split into 2 conical centrifuge bottles and centrifuged for 10 minutes at 1000 RPM and at 3—5°C.

The supernatant liquid is drawn off and 20.4 ml of a pH controlled buffer stabilizer (90 ml SPGA+10 ml 1M phosphate buffer, pH 7.8) is added to one bottle to attain a theoretical cell concentration of $20 \times 10^6$ cells. Another pH controlled buffer stabilizer (90 ml SPGA+10 ml 1M phosphate buffer pH 6.2) is added to the other bottle in the same manner.

The contents of each of the two bottles are dispensed into 30 ml vials and batch sonicated for 2 minutes at approximately 35 watts and a temperature of 3—5°C. After sonication each sample is dispensed into 3 ml vials (1.1 ml fill) and frozen at −70°C for approximately 6 hours. The vials are lyophilized, sealed and labeled.

Stability of the lyophilized powder is evaluated after storage at 37°C for 3 days and reconstitution with distilled water. Results are summarized in Table 3.

TABLE 3
Effect of stabilizer pH on storage stability of lyophilized
Marek's disease vaccine

| Buffer pH | Titer PFU/dose | | Average titer PFU/dose |
|---|---|---|---|
| | Run 1 | Run 2 | |
| 6.2 | 2110 | 2300 | 2205 |
| 7.8 | 7360 | 12310 | 9970 |

3

As the examples illustrate, the vaccine prepared with the pH controlled buffered stabilizer has a significantly higher stability when reconstituted with distilled water than the vaccine prepared with simple diluent stabilizer of the prior art. These examples used SPGA, a stabilizer which already contains phosphate. For instance 200 ml of SPGA is made up of 14.92 gm, sucrose, 0.10 gm $KH_2PO_4$, 0.32 gm $K_2HPO_4$, 0.01 gm Na glutamate, and 2.0 gm albumin. By contrast, the dry weights of these components in 1 vial of our new vaccine product (to be diluted with 200 ml water) is 73.9 mg sucrose, 3.4 mg $KH_2PO_4$, 16.6 mg $K_2HPO_4$, 0.9 mg Na glutamate, and 9.9 mg albumin. We have calculated that as long as a preferred level of about 20 mg of total phosphate buffer is present in a finished vaccine vial, any stabilizer can be used during lyophilization. Obviously calculations can be made by the user. We have further determined that between about 10—60 mg per finished vial of total phosphate buffer can be operably employed. In parts by weight per liter the compositions of the present invention contain from 33 to 135 parts sucrose, from 1.5 to 15 parts $KH_2PO_4$, from 7 to 30 parts $K_2HPO_4$, from 0.4 to 0.17 part monosodium glutamate, and from 4.5 to 18 parts albumin. Within these ranges, the ratio $K_2HPO_4$ to $KH_2PO_4$ should be 10 to 2, and is preferably about 5. Given these figures, adjustments to any commonly used stabilizer system can be made to yield the stabilized vaccine of this invention, having the advantage of being easily diluted for use with distilled water.

**Claims**

1. A composition characterized by the fact that it comprises:

— a lyophilized cell-free turkey herpes virus,
— a herpes virus stabilizer containing at least one member selected from sucrose and monosodium glutamate together with albumin,
— and an amount of phosphate buffer effective to maintain the pH at from 6.8 to 7.2 after reconstitution of the virus with water to use level by addition of substantially 200 volumes of water relative to the pre-lyophilization volume of the vaccine.

2. The composition of claim 1, characterized by the fact that the virus is strain ATCC VR-584.

3. The composition of claim 1 or 2, characterized by the fact that the stabilizer comprises sucrose, phosphate, monosodium glutamate and albumin.

4. An aqueous suspension, characterized by the fact that it comprises the reconstituted composition of claim 1.

5. The suspension of claim 4, characterized by the fact that the virus is strain ATCC VR-584.

6. The suspension of claim 4, characterized by the fact that the stabilizer comprises sucrose, phosphate, monosodium glutamate and albumin.

7. The suspension of claim 6, characterized by the fact that it comprises in parts by weight per 1000 parts water, from 33 to 135 parts sucrose, from 1.5 to 15 parts $KH_2PO_4$, from 7 to 30 parts $K_2HPO_4$, from 0.4 to 0.17 part monosodium glutamate, and from 4.5 to 18 parts albumin.

8. A method of preparing a lyophilized turkey herpes virus capable of being reconstituted with water, characterized by the fact that it comprises lyophilizing the turkey herpes virus in the presence of a herpes virus stabilizer containing at least one member selected from sucrose and monosodium glutamate together with albumin and an amount of a buffer effective to maintain the pH at from 6.8 to 7.2 after reconstitution of the virus with water to use level by addition of substantially 200 volumes of water relative to the prelyophilization volume of the vaccine.

9. A method according to claim 8, characterized by the fact that the virus is strain ATCC VR-584.

**Patentansprüche**

1. Zusammensetzung, dadurch gekennzeichnet, dass sie enthält:

— einen lyophilisierten, zellfreien Truthahn-Herpesvirus,
— einen Herpesvirus-Stabilisator, enthaltend mindestens einen Bestandteil ausgewählt aus Saccharose und Mononatriumglutamat zusammen mit Albumin,
— eine Menge Phosphatpuffer, die nach Rekonstitution des Virus mit Wasser die Anwendungskonzentration durch Zusatz von im wesentlichen 200 Volumenteilen Wasser, bezogen auf das Prälyophilisationsvolumen des Impfstoffs, zur Aufrechterhaltung eines pH-Werts von 6,2 bis 7,2 ausreicht.

2. Zusammensetzung nach Anspruch 1, dadurch gekennzeichnet, dass es sich bei dem Virus um den Stamm ATCC VR-584 handelt.

3. Zusammensetzung nach Anspruch 1 oder 2, dadurch gekennzeichnet, dass der Stabilisator Saccharose, Phosphat, Monoatriumglutamat und Albumin umfasst.

4. Wässrige Suspension, dadurch gekennzeichnet, dass sie die rekonstituierte Zusammensetzung nach Anspruch 1 umfasst.

5. Suspension nach Anspruch 4, dadurch gekennzeichnet, dass es sich bei dem Virus um den Stamm ATCC VR-584 handelt.

**0 008 255**

6. Suspension nach Anspruch 4, dadurch gekennzeichnet, dass der Stabilisator Saccharose, Phosphat, Monoatriumglutamat und Albumin umfasst.

7. Suspension nach Anspruch 6, dadurch gekennzeichnet, dass sie pro 1000 Gewichtsteile Wasser 33 bis 135 Teile Saccharose, 1,5 bis 15 Teile $KH_2PO_4$, 7 bis 30 Teile $K_2HPO_4$, 0,4 bis 0,17 Teile Monoatriumglutamat und 4,5 bis 18 Teile Albumin enthält.

8. Verfahren zur Herstellung eines lyophilisierten Truthahn-Herpesvirus, der zur Rekonstitution mit Wasser geeignet ist, dadurch gekennzeichnet, dass man den Truthahn-Herpesvirus in Gegenwart eines Herpesvirus-Stabilisators, der mindestens einen Bestandteil ausgewählt aus Saccharose und Monoatriumglutamat zusammen mit Albumin und eine Menge eines Puffers, die nach Rekonstitution des Virus mit Wasser auf Anwendungskonzentration durch Zusatz von im wesentlichen 200 Volumenteilen Wasser, bezogen auf das Prälyophilisationsvolumen des Impfstoffs, zur Aufrechterhaltung eines pH-Werts von 6,8 bis 7,2 ausreicht.

9. Verfahren nach Anspruch 8, dadurch gekennzeichnet, dass es sich bei dem Virus um den Stamm ATCC VR-584 handelt.

**Revendications**

1. Composition caractérisé en ce qu'elle contient:

— un virus de l'herpès du dindon exempt de cellules et lyophilisé,
— un stabilisateur de virus de l'herpès contenant au moins un élément choisi entre le saccharose et le glutamate monosodique avec l'albumine, et
— une quantité de tampon phosphaté efficace pour maintenir le pH entre 6,8 et 7,2 après reconstitution du virus avec de l'eau au niveau d'utilisation par addition de pratiquement 200 volumes d'eau par rapport au volume de pré-lyophilisation du vaccin.

2. Composition de la revendication 1, caractérisé en ce que le virus est la souche ATCC VR-584.

3. Composition de l'une des revendications 1 ou 2, caractérisée en ce que le stabilisateur comprend du saccharose, du phosphate, du glutamate monosodique et de l'albumine.

4. Suspension aqueuse caractérisée en ce qu'elle comprend la composition reconstituée de la revendication 1.

5. Suspension de la revendication 4, caractérisée en ce que le virus est la souche ATCC VR-584.

6. Suspension de la revendication 4, caractérisée en ce que le stabilisateur comprend du saccharose, du phosphate, du glutamate monosodique et de l'albumine.

7. Suspension de la revendication 6, caractérisée en ce qu'elle comprend en fractions pondérales pour 1000 parties d'eau, de 33 à 135 parties de saccharose, de 1,5 à 15 parties de $KH_2PO_4$, de 7 à 30 parties de $K_2HPO_4$, de 0,4 à 0,17 parties de glutamate monosodique, et de 4,5 à 18 parties d'albumine.

8. Procédé de préparation d'un virus de l'herpès du dindon lyophilisé pouvant être reconstitué avec de l'eau, caractérisé en ce qu'il comprend la lyophilisation du virus de l'herpès du dindon en présence d'un stabilisateur de virus de l'herpès contenant au moins un élément choisi entre le saccharose et le glutamate monosodique avec de l'albumine et une quantité d'un tampon efficace pour maintenir le pH entre 6,8 et 7,2 après reconstitution du virus avec de l'eau jusqu'au niveau d'utilisation par addition de pratiquement 200 volumes d'eau par rapport au volume de prélyophilisation du vaccin.

9. Procédé selon la revendication 8, caractérisé en ce que le virus est la souche ATCC VR-584.

5